# EUROPEAN PATENT SPECIFICATION

(11) **EP 3 030 216 B1**
(45) Date of publication and mention of the grant of the patent: **18.12.2019**
(21) Application number: 14752764.2
(22) Date of filing: 08.08.2014
(51) Int. Cl.: A61K 8/31, A61K 8/37, A61K 8/49, A61Q 19/00

(54) **SKIN CARE COMPOSITIONS HAVING CYCLIC DIESTERS AND METHODS THEREOF**
HAUTPFLEGEZUSAMMENSETZUNGEN MIT CYCLISCHEN DIESTERN UND VERFAHREN DAFÜR
COMPOSITIONS DE SOINS DE LA PEAU AYANT DES DIESTERS CYCLIQUES ET MÉTHODES ASSOCIÉES

(30) Priority: 09.08.2013 US 201361864172 P
(43) Date of publication of application: 15.06.2016
(73) Proprietor: The Chemours Company FC, LLC, Wilmington DE 19801 (US)
(72) Inventor: CAHILL, William R., Jr., Hockessin, Delaware 19707 (US); BURCH, Robert Ray, Exton, Pennsylvania 19341 (US); HORSAGER, Jeffrey Jon, Edina, Minnesota 55424 (US); REISACK, Jessica Linda, Middletown, Delaware 19709 (US); ALTLAND, Jennifer Marie, Lancaster, Pennsylvania 17603 (US)
(74) Representative: Morf, Jan Stefan
(86) International application number: PCT/US2014/050303
(87) International publication number: WO 2015/021362

(56) References cited:
- JP-A- 2002 179 528
- US-A- 5 610 199
- US-A1- 2003 017 130
- US-A1- 2012 149 920

## Description

### FIELD OF THE INVENTION

The present invention generally relates to topical skin care compositions having at least one cyclic diester. More specifically, the present invention relates to novel topical skin care compositions having at least one cyclic diester of an alpha hydroxy acid, and at least one polar non-aqueous solvent.

### BACKGROUND OF THE INVENTION

Alpha hydroxy acids ("AHAs") are known to be useful in skin care compositions for treating various skin conditions, including rhytids (i.e., wrinkles), xeroderma (i.e., dry skin), hyperkeratosis, ichthyosis, and discoloration. Specifically, AHAs that are short chain carboxylic acids, such as glycolic acid and lactic acid, are preferred in cosmetic compositions due to the AHAs ability to penetrate skin. In particular, the bioavailability of short chain AHAs stimulates cellular activity in the epidermis and dermis, as well as increases desquamation of the outer layers of the epidermis to help alleviate and treat the skin conditions above. Furthermore, short chain AHAs can aid and stimulate collagen synthesis, which further helps reduce rhytids, while improving skin elasticity and firmness.

However, a major problem with using AHAs in skin care compositions is the fact that AHAs are acids, which can lead to skin irritation. And while using AHAs for their acidic properties may be desirable in certain applications, such as for skin peel applications, the acidity of AHAs can have detrimental and undesirable effects for more daily and routine applications.

In order to reduce the irritation associated with using AHAs in skin care compositions, other compounds can be added in an attempt to make the overall skin care composition less acidic. For example, U.S. Patents 5,886,042 and 5,385,938 discuss adding an amphoteric or pseudoamphoteric compound with the AHAs to try and raise the overall pH of the cosmetic composition. However, not only do these compositions require an additional component, such as amino acids and imidazoline compounds, which may not be desirable for a particular composition or use, but this strategy also does not address the underlying issue regarding the acidity of the AHAs. Rather, by attempting to balance the AHA with another compound, the acidity of the AHA is merely being masked and not reduced. By not addressing the problematic acidity of the AHAs, skin irritation and intolerability can persist, especially in users with sensitive skin.

In addition to problems associated with the acidity of AHAs, skin care compositions in the field generally have problems sustaining relatively long-term stability, while also allowing sufficient penetration of the active ingredient into the skin. Stability problems can occur based on a variety of environmental factors, including changes in temperature and humidity during processing, shipping, storage, and use, as well as chemical factors within the compositions, including the miscibility or homogeneity of the various components. In this respect, less stable compositions can be more acidic and can potentially become more acidic over time due to masking components becoming diminished or separating out of the compositions, which further exacerbates the irritability of the compositions.

Accordingly, there remains a need in the art for skin care compositions that have reduced acidity and potential irritability, while also having sufficient stability and penetration properties. As such, there remains a need in the art for skin care compositions having at least one cyclic diester, such as at least one cyclic diester of an AHA. Moreover, there remains a need in the art for skin care compositions having the aforementioned cyclic diester and at least one polar non-aqueous solvent.

US patent application US 2003/0017130 A1 discloses preventive as well as therapeutic treatment to alleviate cosmetic conditions and symptoms of dermatologic disorders with amphoteric compositions containing alpha hydroxyacids, alpha ketoacids, related compounds or polymeric forms of hydroxyacids.

### SUMMARY OF THE INVENTION

The present invention generally relates to the non-therapeutic use of a composition as a skin care composition in accordance with claim 1.

### BRIEF DESCRIPTION OF THE DRAWINGS

Fig. 1 represents the relative skin penetration of two exemplary skin care compositions versus a control of water and a composition not having at least one polar non-aqueous solvent, in which the layers are along the x-axis and the absorbance at 1766 cm-1 is along the y-axis.
Fig. 2 represents the relative skin penetration of two exemplary skin care compositions versus a control of water, in which the layers are along the x-axis and the absorbance at 1766 cm-1 is along the y-axis.
Fig. 3 represents the relative skin penetration of two exemplary skin care compositions versus a control of water, in which the layers are along the x-axis and the absorbance at 1766 cm-1 is along the y-axis.
Fig. 4 represents the relative skin penetration of two exemplary skin care compositions versus three compositions not having at least one polar non-aqueous solvent, in which the layers are along the x-axis and the absorbance at 1766 cm-1 is along the y-axis.

### DETAILED DESCRIPTION OF THE INVENTION

### Definitions:

The term and phrases "invention," "present invention," "instant invention," and similar terms and phrases as used herein are non-limiting and are not intended to limit the present subject matter to any single embodiment, but rather encompass all possible embodiments as described.

As used herein, the term "about" means within 15% of the reported numerical value; in another embodiment, the term "about" means within 10% of the reported numerical value.

As used herein, the term "skin care composition," "cosmetic," "cosmetic composition," and similar terms, including plural terms, can be used interchangeably. Specifically, the term skin care composition includes compositions that can be rubbed, poured, sprinkled, or sprayed on, introduced into, or otherwise applied to the human body, including skin, or any part thereof for cleansing, beautifying, promoting attractiveness, or altering the appearance, as well as includes compositions intended for use as a component of another cosmetic. The term "skin care composition" and other terms above do not exclude soap, and specifically includes topical compositions for application to human skin.

As used herein, the terms "a," or in the alternative "the" when referencing a component already disclosed, and "at least one" shall have the same meaning and can be used interchangeably.

As used herein, all weight percentages (wt. %) are based on the total wt. % of the skin care composition, unless otherwise specified. Additionally, all composition percentages are based on totals equal to 100 wt. %, unless otherwise specified.

### Skin care compositions:

The skin care compositions herein provide an alternative to other compositions having AHAs, while at the same time providing stable compositions having reduced acidity, including in preferred embodiments having low acidity, and good skin penetration.

As discussed above, while the use of AHAs has increased in the cosmetic industry due to the beneficial effects AHAs have on skin, a major drawback with using AHAs is the acidic nature of the compounds. For instance, glycolic acid and lactic acid, which are C₂ and C₃ AHAs respectively, both have pKa values less than 4. *See, e.g.,* W.M. Haynes, CRC Handbook of Chemistry and Physics 5-94 to 5-95 (94th ed. 2013). This relatively high acidity can cause irritation and discomfort to the user, especially users with sensitive skin.

Surprisingly, it has been found that by using at least one cyclic diester of an alpha hydroxy acid, and at least one polar non-aqueous solvent, skin care compositions can be formed having very low acidity and good stability, while also allowing the cyclic diester to penetrate skin. Since the present skin care compositions have such low acidity, the irritation and discomfort associated with traditional compositions containing AHAs can be minimized or prevented. Additionally, since the instant invention provides a way for the cyclic diester to penetrate the skin upon topical application, the benefits associated with the cyclic diesters and corresponding AHAs can be obtained by the user.

In order to reduce the irritation and discomfort associated with compositions having AHAs, generally in preferred embodiments, the skin care compositions can have a pH of 5.5 to 8, more preferably 6 to 8, and even more preferably the skin care compositions can have a pH of about 7. Alternatively, the skin care compositions can be formulated with other components to have a lower pH, including a pH of 3 to 8, more preferably 3.5 to 7, and even more preferably 3.8 to 4.2.

Moreover, in addition to the pH, in preferred embodiments the skin care compositions can have an acidity of 10 wt. % or less, more preferably 8 wt. % or less, and even more preferably 5 wt. % or less at about 20 °C. The acidity of the skin care compositions can be determined by the amount of cyclic ester in the skin care composition that has converted into another form and is not in the cyclic form. Specifically, the acidity of the skin care compositions can be determined by the amount of cyclic ester that has converted into an acidic, non-cyclic dimer form and the amount of cyclic ester that has converted into the corresponding free form AHAs, as well as any AHAs that may have been added to the compositions.

The current skin care compositions also allow cyclic diesters of an alpha hydroxy acid, or mixtures of cyclic diesters, to penetrate skin. In particular embodiments, the skin care compositions can improve the penetration of at least one cyclic diester of an alpha hydroxy acid through at least one layer of skin by at least 30%, preferably at least 40%, and more preferably at least 50% versus skin care compositions not having at least one polar non-aqueous solvent. Furthermore, in certain embodiments, the skin care compositions can provide an absorbance of at least 0.15, preferably at least 0.17, more preferably at least 0.2, and even more preferably at least 0.25 for at least one skin layer determined by attenuated total reflectance (ATR)-FTIR spectroscopy at 1766 cm-1. In particularly preferred embodiments, the skin care compositions can provide an absorbance of 0.15 to 0.5, preferably 0.17 to 0.4, and more preferably 0.2 to 0.35 for at least one skin layer determined by attenuated total reflectance (ATR)-FTIR spectroscopy at 1766 cm-1.

### Cyclic diesters of an alpha hydroxy acid:

The cyclic diester that can be used in the invention can be any cyclic diester of any alpha hydroxy acid. In preferred embodiments, the cyclic diester is a C₄-C₈ diester, which can be formed from the dimerization of two C₂-C₄ AHAs. In this respect, the cyclic diester can be a cyclic dimer of two identical AHAs, or a combination of two different AHAs. For example, the cyclic diester can be formed by two glycolic acid molecules resulting in glycolide, two lactic acid molecules to form lactide, or one glycolic acid and one lactic acid molecule. Additionally, all isomers of the AHAs are encompassed, including all isomers of lactide, such as the D- and L- isomers.

In certain embodiments, the cyclic diester can have the following formula: 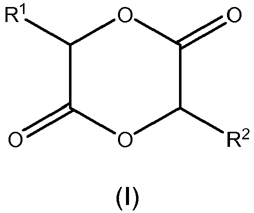 wherein R¹ is hydrogen or a C₁-C₂ alkyl, alkenyl, or alkynyl; and R² is hydrogen or a C₁-C₂ alkyl, alkenyl, or alkynyl. In preferred embodiments, the cyclic diester can have formula (I) wherein R¹ is hydrogen, methyl, or ethyl; and R² is hydrogen, methyl, or ethyl. In a more preferred embodiment, the cyclic diester can be glycolide or lactide, in which R¹ and R² are both hydrogen or methyl, respectively.

Additionally, while the cyclic diester can be in a single chemical form, for example, glycolide or lactide, the skin care compositions of the instant invention can also have mixtures of various cyclic diesters. In particular, the skin care compositions can have mixtures of C₄-C₈ diesters, including but not limited to mixtures of glycolide and lactide.

With respect to the amount of cyclic diester in current skin care compositions, the compositions comprise 0.1 to 30 wt. % of at least one cyclic diester of an alpha hydroxy acid. In preferred embodiments, the skin care compositions can comprise 0.1 to 20 wt. %, including 0.5 to 10 wt. %, and more preferably 0.5 to 5 wt. %, including 1 to 5 wt. %, of at least one cyclic diester. In specifically preferred embodiments, the skin care compositions can comprise 0.1 to 20 wt. %, including 0.5 to 10 wt. %, and more preferably 0.5 to 5 wt. %, including 1 to 5 wt. %, of glycolide, lactide, or combinations thereof. In embodiments having mixtures of cyclic diesters, preferably the skin care compositions can have 0.1 to 10 wt. %, more preferably 0.5 to 5 wt. %, including 1 to 5 wt. %, of glycolide. In other embodiments having mixtures of cyclic diesters, preferably the skin care compositions can have 0.1 to 10 wt. %, more preferably 0.5 to 5 wt. %, including 1 to 5 wt. %, of lactide.

### Polar non-aqueous solvents:

Various polar non-aqueous solvents can be used in the instant skin care compositions. Specifically, at least one polar non-aqueous solvent in the instant skin care compositions should be able to dissolve a cyclic diester of an alpha hydroxy acid to some degree. In this respect, in preferred embodiments, the polar non-aqueous solvent can dissolve at least 1 wt. %, more preferably at least 5 wt. %, and even more preferably at least 10 wt. % of the cyclic diester based on the weight of the solvent in the skin care composition. In particularly preferred embodiments, at least one polar non-aqueous solvent can dissolve 1 to 20 wt. %, more preferably 5 to 20 wt. %, and even more preferably 10 to 20 wt. % of the cyclic diester based on the weight of the solvent in the skin care composition.

Moreover, the polar non-aqueous solvent can be any organic solvent. In certain embodiments, the polar non-aqueous solvent can be a polar C₁-C₁₅ solvent, more preferably the solvent can be a polar C₁-C₁₀ solvent, and even more preferably the solvent can be a polar C₂-C₁₀ solvent. Specifically, the polar non-aqueous solvent has a polarity of 5-20 as defined in Hansen Solubility Parameters: A User's Handbook (2nd ed. 2007). In certain preferred embodiments, the polar non-aqueous solvent can have a polarity of at least 8, as defined in C.M. Hansen, Hansen Solubility Parameters: A User's Handbook (2nd ed. 2007) ("Hansen"). The polar non-aqueous solvent has a polarity of 5 to 20, and preferably 8 to 18, as defined in Hansen.

In addition to polarity, the non-aqueous solvents can have a certain hydrogen bonding potential, as defined in Hansen. In preferred embodiments, the polar non-aqueous solvent can have a hydrogen bonding potential of at least 5 up to 25, and more preferably at least 5 up to 20, as defined in Hansen. In particularly preferred embodiments, the polar non-aqueous solvent can have a hydrogen bonding potential of at least 5 up to 20, and a polarity of at least 5 up to 20, as defined in Hansen. Even more preferred embodiments can have a hydrogen bonding potential of 6 to 10, and can have a polarity of 7 to 11, as defined in Hansen.

With respect to the chemical structure, the polar non-aqueous solvent can be substituted or unsubstituted, and can be linear, branched, or cyclic, including bicyclic, aromatic, or both. If the solvent is cyclic, including bicyclic, aromatic, or both, the solvent can have at least one heteroatom in the cyclic structure, including but not limited to oxygen, nitrogen, or combinations thereof. Additionally, while the polar non-aqueous solvent is not limited to having any particular functional group(s), in preferred embodiments, the polar non-aqueous solvent can have at least one carbonyl, ether, alcohol, amide, amine, imine, cyanate, isocyanate, nitrile, isonitrile, and combinations thereof.

In certain embodiments, the skin care compositions can have at least one polar non-aqueous solvent of formula (II): 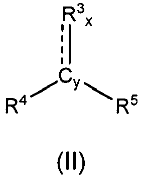 wherein R³ is hydrogen, oxygen, ether, or ester; R⁴ is hydrogen or an alkyl, alkenyl, alkynyl, or alkyl hydroxy group; R⁵ is nitrogen, an alkyl, hydroxyl ether, ester, amide, or amine; R⁶ is an alkyl, ether, ester, amide, amine, or combinations thereof; and x is 0 or 1; with the proviso that if R⁵ is nitrogen and forms a nitrile group, then x is 0, and if R³ is oxygen, then C_{y} and R³ form a carbonyl; wherein R⁴ and R⁵ can join to form a cyclic or bicyclic structure including an aromatic structure, which can include heteroatoms and can be optionally substituted with at least one R⁶ group. If formula (II) has more than one R⁶ group, the R⁶ groups can be the same or different. Preferably, R³ is oxygen to form a carbonyl with C_{y}, or a C₁-C₅ alkoxy or aryloxy group; R⁴ is hydrogen or a C₁-C₅ alkyl; and R⁵ is nitrogen, a C₁-C₅ alkyl, a C₁-C₅ ether, a C₁-C₅ ester, or an amide; with the proviso that if R⁵ is nitrogen and forms a nitrile group, then x is 0.

Alternatively, in other preferred embodiments, the skin care compositions can have at least one polar non-aqueous solvent of formula (III), formula (IV), or mixtures thereof: 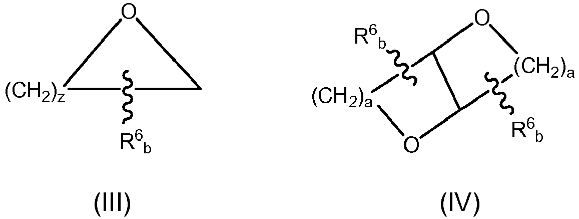 wherein R⁶ is an alkyl, ether, ester, amide, amine, or combinations thereof; a is 1 to 3; b is 1 to 5; and z is 1 to 3. Preferably, R⁶ is a C₁-C₅ alkoxy group, C₁-C₅ aryloxy group, or combinations thereof, and more preferably R⁶ is a methoxy, ethoxy, or combinations thereof. Further, in preferred embodiments, b is 1 to 4, and more preferably 1 to 3.

In particularly preferred embodiments, the polar non-aqueous solvent can be polar C₁-C₁₀ alcohols, including diols, ketones, esters, ethers, cyclic ethers, amides, nitriles, and mixtures thereof, and specifically includes polar C₁-C₅ linear or branched, substituted or unsubstituted ketones, esters, ethers, amides, nitriles, and mixtures thereof. Additionally, the polar non-aqueous solvent specifically includes polar C₄-C₁₀ cyclic ethers, cyclic ketones, and mixtures thereof, which can be substituted or unsubstituted. Moreover, the polar non-aqueous solvent can be a mixture of solvents of formula (II), (III), and/or (IV). In particular embodiments, the non-aqueous solvent can be blends of the polar C₁-C₅ linear or branched, substituted or unsubstituted ketones, esters, ethers, amides, nitriles, and mixtures thereof, with the polar, substituted or unsubstituted C₄-C₁₀ cyclic ethers, cyclic ketones, and mixtures thereof.

Non-limiting examples of particularly preferred embodiments of the polar non-aqueous solvent can include dimethylacetamide, acetonitrile, ethyl acetate, tetrahydrofuran, dimethylformamide, methyl ethyl ketone, cyclohexanone, isosorbide dimethyl ether (also known as "dimethyl isosorbide"), methanol, ethanol, propanol, isopropanol, propylene glycol, and mixtures thereof.

The current skin care compositions comprises 5 to 30 wt. % of at least one polar non-aqueous solvent, and preferably can comprise 10 to 30 wt. %, more preferably 10 to 20 wt. %, of the polar non-aqueous solvent. Alternatively, the skin care composition can comprise 5 to 25 wt. %, and more preferably 7.5 to 15 wt. % of the polar non-aqueous solvent. As discussed, the polar non-aqueous solvent can include mixtures of solvents, the mixtures having a final weight percent according to the ranges above based on the total weight percentage of the composition.

### Bases:

The bases useful in the instant skin care compositions can include bases generally used in cosmetic and skin care compositions. As opposed to the polar non-aqueous solvent, preferably the base can be non-polar, or have a relatively low polarity. More specifically, the base has a polarity of 5 or less, and preferably 2.5 or less, as defined in Hansen. In specific embodiments, the base can be non-polar, or have a polarity of 1 or less, as defined in Hansen.

In addition to having a relatively low polarity, or being non-polar, the base can generally be hydrophobic and can have a relatively low water content. Specifically, the base can have less than 1 wt. % of water, more preferably less than 0.5 wt. % of water, and in particularly preferred embodiments, the base can have less than 0.1 wt. % of water.

In certain embodiments, the base can be a long chain, saturated or unsaturated, aliphatic hydrocarbon, including a cyclic aliphatic hydrocarbon, and mixtures thereof. More specifically, in certain preferred embodiments, the base can comprise at least one straight or branched C₁₀ or higher long chain alkane, alkene, or alkyne, and preferably comprises at least one C₁₀-C₅₀ straight or branched alkane, alkene, or alkyne. In particularly preferred embodiments, the base can have at least one C₁₅-C₄₀, more preferably at least one C₂₅-C₃₅, straight or branched alkane, alkene, or alkyne. Alternatively, the base can comprise at least one cyclic aliphatic hydrocarbon, optionally substituted with at least one straight or branched C₈ or higher, preferably at least one C₈-C₅₀, more preferably at least one C₁₀-C₃₅ straight or branched alkane, alkene, or alkyne. Additionally, the base can be mixtures of the straight or branched C₁₀ or higher long chain alkane, alkene, or alkyne, including the preferred C₁₀-C₅₀, and more preferred C₁₅-C₄₀, and even more preferred C₂₅-C₃₅, straight or branched alkane, alkene, or alkyne, with the cyclic aliphatic hydrocarbon, optionally substituted with at least one straight or branched C₈ or higher, preferably at least one C₈-C₅₀, more preferably at least one C₁₀-C₃₅, straight or branched alkane, alkene, or alkyne.

Furthermore, the base can be oleaginous, a wax, or both. In this respect, the base can have a melting point greater than 25 °C, or in alternative embodiments, the base can have a melting point of 25 °C or less.

In specifically preferred, non-limiting embodiments, the base can be petroleum jelly, petrolatum, paraffin wax, mineral oil, and combinations thereof. The skin care compositions comprise at least 40 wt. % of at least one base, and preferably can comprise at least 45 wt. %, more preferably at least 60 wt. %, of at least one base. In certain preferred embodiments, the skin care compositions can comprise at least 80 wt. % of at least one base.

### Other components:

Other active cosmetic compounds, active pharmaceutical compounds, or mixtures thereof, may be included in the instant skin care compositions. Non-limiting examples can include compounds that improve or eradicate age spots, keratosis, and wrinkles; exfoliates, analgesics; anesthetics; antiacne agents; antibacterials; antiyeast agents; antifungal agents; antiviral agents; antidandruff agents; antidermatitis agents; antipruritic agents; antiemetics; antiinflammatory agents; antihyperkeratolytic agents; moisturizers; antiperspirants; antipsoriatic agents; antiseborrheic agents; hair conditioners and hair treatment agents; antiaging agents; antiasthmatic agents and bronchodilators; sunscreen agents; antihistamine agents; skin lightening agents; depigmenting agents; vitamins; corticosteroids; tanning agents; hormones; retinoids; topical cardiovascular agents, and other dermatologicals.

Generally, other active components may be present up to 15 wt. %, preferably up to 10 wt. %, and more preferably up to 5 wt. %. More specifically, the instant skin care compositions can have 0 to 15 wt. %, preferably 0 to 10 wt. %, and even more preferably 0 to 5 wt. % of additional active components.

Moreover, while the skin care compositions can have water present, the compositions have less than 1 wt. %, preferably 0.5 wt. % or less, more preferably 0.25 wt. % or less, and most preferably 0.1 wt. % or less of water. The skin care compositions can be relatively anhydrous with no water present up to less than 1 wt. %, preferably less than 0.5 wt. %, more preferably less than 0.1 wt. %, including residual water. In this respect, the skin care compositions can have at least one water scavenger, including, but not limited to fumed silica, aluminosilicate, aluminum silicate, including magnesium aluminum silicate, aluminum starch octenylsuccinate, and combinations thereof. The water scavenger can be present up to 15 wt. %, preferably up to 10 wt. %, and more preferably up to 5 wt. %.

Various surfactants, emulsifiers, gelling agents, stabilizers, plasticizers, rheology agents, and combinations thereof, can be added to the instant skin care compositions. Specifically, as a non-limiting example, at least one surfactant can be added to homogenize the skin care compositions, as well as increase the miscibility between the non-aqueous solvent and base. The surfactants, emulsifiers, gelling agents, stabilizers, plasticizers, rheology agents, and combinations thereof, can be present up to 15 wt. %, and preferably up to 10 wt. %.

In addition to the cyclic diesters, the skin care compositions can have AHAs present. While the amount of the AHAs can generally be less than the amount of the cyclic diesters, the instant compositions can have up to 5 wt. % of AHAs present, more preferably up to 2.5 wt. % of AHAs present, and even more preferably up to 1 wt. % of AHAs present. Specifically preferred AHAs can include glycolic acid, lactic acid, or combinations thereof. Further, if AHAs are present in the skin care compositions, the AHAs can be added to the compositions in addition to the cyclic diesters, or the AHAs can be produced by the hydrolyzation of the cyclic diesters of the alpha hydroxy acids. However, in order to minimize the potential irritability of the skin care compositions, and within the general pH and acidity ranges indicated above for the compositions, the amount of AHAs present, either as an added component or from hydrolysis of the cyclic diester, should be relatively low and in accordance with the ranges above.

Besides the other active compounds, other inactive compounds such as, but not limited to colorants, fragrances, abrasive compounds, including silica dioxide, polymeric resins, clays, and combinations thereof can be added to the skin care compositions. Generally, the inactive compounds may be present up to 5 wt. %, preferably up to 2.5 wt. %, and more preferably up to 1 wt. %. More specifically, the instant skin care compositions can have 0 to 5 wt. %, preferably 0 to 2.5 wt. %, and even more preferably 0 to 1 wt. % of the inactive compounds.

### Method of making the skin care compositions:

The current skin care compositions can be made in a variety of ways, including in a continuous process or in a batch process. For example, as a non-limiting example, all of the components, including the cyclic diester of an alpha hydroxy acid, the polar non-aqueous solvent, and the aliphatic base can be added together at the same time in the amounts previously indicated. Alternatively, as another non-limiting example, certain components can be added together first with other components added subsequently.

In certain embodiments, the skin care compositions can be made by adding at least one cyclic diester of an alpha hydroxy acid, at least one polar non-aqueous solvent, and at least one aliphatic base together to form a substantially homogenous mixture. In this respect, the substantially homogenous mixture can have less than 5 wt. %, more preferably less than 1 wt. %, even more preferably less than 0.5 wt. %, and most preferably less than 0.1 wt. % of the cyclic diester of an alpha hydroxy acid, the polar non-aqueous solvent, and/or the aliphatic base in a separate phase than the mixture.

In addition to the method used to add together the various components of the skin care compositions, including a cyclic diester of an alpha hydroxy acid, at least one polar non-aqueous solvent, and at least one aliphatic base, the components can be heated, including heating the components to make a substantially homogenous mixture. Specifically, as a non-limiting example, the cyclic diester, polar non-aqueous solvent, and aliphatic base can be heated to the melting point of the aliphatic base. In other non-limiting embodiments, the aliphatic base can be heated to the melting point of the base, and then the aliphatic base can be added to a mixture of the cyclic diester and solvent.

### EXAMPLES

The following examples are illustrative of preferred skin care compositions and are not intended to be limitations thereon. All numerical values given are in weight percentage, and all product composition percentages are based on totals equal to 100% by weight, unless otherwise specified.

### Test Methods:

Acidity Test: The acidity test determined the amount of acid formation in a tested example and was performed by titration. A Mettler Toledo DL58 Titrator apparatus equipped with a Mettler Toledo DM140-SC electrode was used to perform the potentiometric titration to determine the acidity of the tested example. The titrant consisted of 71 wt. % of toluene, 19 wt. % of methanol, and 10 wt. % of 1N tetrabutylammonium hydroxide. The titration was run at room temperature, which means at about 20 °C. The acid formation results are reported as the weight percentage of acid from the conversion of glycolide or lactide into the acidic, non-cyclic dimer form and glycolic acid or lactic acid, respectively. The weight percentage of acid is calculated based on the molecular weight of glycolic acid or lactic acid, respectively.

Aging Test: The aging test simulates storage stability. Each example tested was split into a room temperature sample ("R.T.") and an aged sample ("Aged"). The aged samples were placed in a VWR 1410 oven at 54 °C for fourteen (14) days, after which the aged samples were removed from the oven and tested for the amount of acid formation using the Acidity Test. The room temperature samples were tested without oven aging for the amount of acid formation using the Acidity Test.

Skin Penetration Test: The skin penetration test determined the deposition, penetration, and conversion of the tested cyclic ester into the corresponding alpha hydroxy acids in the outer layers of *ex vivo* porcine skin by using attenuated total reflectance (ATR)-FTIR spectroscopy and tape stripping. The resulting absorbance reported was determined at 1766 cm-1. The FTIR spectrometer used was a Nicolet 700 FT-IR from Thermo Electron Corporation. Scotch® Magic™ Tape was used for the tape stripping, which is available from 3M, St. Paul, Minnesota, USA.

### Procedure:

Step 1: A sample of porcine skin was washed with water;
Step 2: The porcine skin was then scanned using ATR-FTIR as the control ("Control Layer");
Step 3: Each composition that was tested, as indicated below in the tables, was then applied to the porcine skin and allowed to stand for two (2) hours at 34°C;
Step 4: After two (2) hours, the excess composition was removed from the surface of the porcine skin;
Step 5: The porcine skin was then scanned by ATR-FTIR as the surface ("Surface Layer");
Step 6: A strip of tape was then evenly applied to the treated area on the porcine skin;
Step 7: The strip of tape was then removed from the porcine skin;
Step 8: The porcine skin was then scanned by ATR-FTIR monitoring the absorbance at 1766 cm-1, and recording the result as "Skin Layer 1"; and
Step 9: Steps 3 - 8 were repeated nine (9) more times, which each subsequent scanned skin layer numbered accordingly. In particular, skin layers 1-10 correspond to the number of times a strip of tape was applied to and removed from the skin sample, and the skin sample was scanned by ATR-FTIR.

### Materials:

"DMI" is dimethyl isosorbide, also known as isosorbide dimethyl ether, which is available from Grant Industries Inc., Elmwood Park, New Jersey, USA.

"Glycolic Acid" is DuPont™ Glypure® glycolic acid, which is available from E. I. Du Pont de Nemours & Co. Inc., Wilmington, Delaware, USA.

"Glycolide" is a C₄ cyclic diester formed from glycolic acid having a purity of at least 99 wt. %.

"Lactic Acid" is Lactic Acid, which is available from JM Baker, Japan.

"Lactide" is L-Lactide, which is available from TCI Tokyo Chemical Industry Co., Ltd, Tokyo, Japan.

"Mineral Oil" is mineral oil, which is available from Roberts, Eatontown, New Jersey, USA, or CVS, Woonsocket, Rhode Island, USA.

"Petroleum Jelly" is Ultima White Pet., USP, which is available from Calumet Penreco, Karns City, Pennsylvania, USA.

"Water" is deionized water.

### Example 1:

A 100 g skin care composition was prepared by adding 85 g of petroleum jelly, 10 g of dimethyl isosorbide, and 5 g of glycolide into a mixing container and heating the components to 50 °C. When the petroleum jelly started to liquefy, the petroleum jelly, dimethyl isosorbide, and glycolide were mixed together at 50 °C using a Lightnin overhead mixer equipped with a 3-blade tilted paddle until a homogenous mixture was obtained. After a homogenous mixture was formed at 50 °C, the heat was removed and mixing continued until the homogenous mixture became too viscous to stir.

The homogenous mixture was then tested for acidity, the results of which are reported in Table 1.

### Comparative Example 1:

A 100 g comparative composition was prepared in the same manner as Example 1, with the exceptions that 1 g of water was added to the petroleum jelly, dimethyl isosorbide, and glycolide, and 84 g of petroleum jelly was used.

### Comparative Example 2:

A 100 g comparative composition was prepared in the same manner as Example 1, with the exceptions that 5 g of water was added to the petroleum jelly, dimethyl isosorbide, and glycolide, and 80 g of petroleum jelly was used.

### Comparative Example 3:

A 100 g comparative composition was prepared in the same manner as Example 1, with the exception that 5 g of glycolic acid was used instead of 5 g of glycolide.

### Comparative Example 4:

A 100 g comparative composition was prepared in the same manner as Comparative Example 1, with the exception that 5 g of glycolic acid was used instead of 5 g of glycolide.

### Comparative Example 5:

A 100 g comparative composition was prepared in the same manner as Comparative Example 2, with the exception that 5 g of glycolic acid was used instead of 5 g of glycolide.

**Table 1**

| Ingredient (wt. %) | Example 1 | Comp. Ex. 1 | Comp. Ex. 2 | Comp. Ex. 3 | Comp. Ex. 4 | Comp. Ex. 5 |
|---|---|---|---|---|---|---|
| DMI | 10 | 10 | 10 | 10 | 10 | 10 |
| Petroleum Jelly | 85 | 84 | 80 | 85 | 84 | 80 |
| Glycolide | 5 | 5 | 5 | - | - | - |
| Glycolic Acid | - | - | - | 5 | 5 | 5 |
| Water | 1 | 1 | 5 | - | 1 | 5 |
| | | | | | | |
| R.T. - Acid Formation % | 7.1 | 62 | 79 | 106 | 91 | 96 |

### Comparative Example 6:

A 100 g comparative composition was prepared by adding 66 g of petroleum jelly, 32 g of mineral oil, and 2 g of glycolide into a mixing container and heating the components to 50 °C. When the petroleum jelly started to liquefy, the petroleum jelly, mineral oil, and glycolide were mixed together at 50 °C using a Lightnin overhead mixer equipped with a 3-blade tilted paddle until a homogenous mixture was obtained. After a homogenous mixture was formed at 50 °C, the heat was removed and mixing continued until the homogenous mixture became too viscous to stir.

The homogenous mixture was then tested for acidity, the results of which are reported in Table 2.

### Comparative Example 7:

A 100 g comparative composition was prepared in the same manner as Comparative Example 6, with the exceptions that 1 g of water was added to the petroleum jelly, mineral oil, and glycolide, and 65 g of petroleum jelly was used.

### Comparative Example 8:

A 100 g comparative composition was prepared in the same manner as Comparative Example 6, with the exceptions that 5 g of water was added to the petroleum jelly, mineral oil, and glycolide, and 61 g of petroleum jelly was used.

**Table 2**

| Ingredient (wt. %) | Comp. Ex. 6 | Comp. Ex. 7 | Comp. Ex. 8 |
|---|---|---|---|
| Petroleum Jelly | 66 | 65 | 61 |
| Mineral oil | 32 | 32 | 32 |
| Water | - | 1 | 5 |
| Glycolide | 2 | 2 | 2 |
| | | | |
| R.T. - Acid Formation % | 1.4 | 85 | 105 |

### Example 2:

A 100 g skin care composition was prepared in the same manner as Example 1. The homogenous mixture was then tested for acidity, the results of which are reported in Table 3.

### Comparative Example 9:

A 100 g comparative composition was prepared in the same manner as Comparative Example 1.

### Comparative Example 10:

A 100 g comparative composition was prepared in the same manner as Comparative Example 2.

### Comparative Example 11:

A 100 g comparative composition was prepared in the same manner as Comparative Example 3.

### Comparative Example 12:

A 100 g comparative composition was prepared in the same manner as Comparative Example 4.

### Comparative Example 13:

A 100 g comparative composition was prepared in the same manner as Comparative Example 5.

### Example 3:

A 100 g skin care composition was prepared in the same manner as Example 1, with the exceptions that 75 g of petroleum jelly and 15 g of glycolide were used instead of 85 g and 5 g, respectively.

### Comparative Example 14:

A 100 g comparative composition was prepared in the same manner as Example 3, with the exception that 15 g of glycolic acid was used instead of 15 g of glycolide.

**Table 3**

| Ingredient (wt. %) | Example 2 | Comp. Ex. 9 | Comp. Ex. 10 | Comp. Ex. 11 | Comp. Ex. 12 | Comp. Ex. 13 | Example 3 | Comp. Ex. 14 |
|---|---|---|---|---|---|---|---|---|
| DMI | 10 | 10 | 10 | 10 | 10 | 10 | 10 | 10 |
| Petroleum Jelly | 85 | 84 | 80 | 85 | 84 | 80 | 75 | 75 |
| Glycolide | 5 | 5 | 5 | - | - | - | 15 | - |
| Glycolic Acid | - | - | - | 5 | 5 | 5 | - | 15 |
| Water | 1 | 1 | 5 | - | 1 | 5 | - | - |
| | | | | | | | | |
| R.T. - Acid Formation % | 7.1 | 61.7 | 79.3 | 106.7 | 90.7 | 96.5 | 3.6 | 15.3 |
| Aged - Acid Formation % | 2.4 | 59.0 | 109.6 | 67.9 | 73.0 | 80.7 | 2.2 | 56.1 |

### Example 4:

A 100 g skin care composition was prepared in the same manner as Example 1, with the exception that 5 g of lactide was used instead of 5 g of glycolide. The homogenous mixture was then tested for acidity, the results of which are reported in Table 4.

### Comparative Example 15:

A 100 g comparative composition was prepared in the same manner as Comparative Example 1, with the exception that 5 g of lactide was used instead of 5 g of glycolide.

### Comparative Example 16:

A 100 g comparative composition was prepared in the same manner as Comparative Example 2, with the exception that 5 g of lactide was used instead of 5 g of glycolide.

### Example 5:

A 100 g skin care composition was prepared in the same manner as Example 3, with the exception that 15 g of lactide was used instead of 15 g of glycolide. The homogenous mixture was then tested for acidity, the results of which are reported in Table 4.

### Comparative Example 17:

A 100 g comparative composition was prepared in the same manner as Comparative Example 14, with the exception that 15 g of lactic acid was used instead of 15 g of glycolic acid.

**Table 4**

| Ingredient (wt. %) | Example 4 | Comp. Ex. 15 | Comp. Ex. 16 | Example 5 | Comp. Ex. 17 |
|---|---|---|---|---|---|
| DMI | 10 | 10 | 10 | 10 | 10 |
| Petroleum Jelly | 85 | 84 | 80 | 75 | 75 |
| Lactide | 5 | 5 | 5 | 15 | - |
| Lactic Acid | - | - | - | - | 15 |
| Water | - | 1 | 5 | - | - |
| | | | | | |
| R.T. - Acid Formation % | 2.5 | 61.7 | 79.3 | 1.5 | 88.5 |
| Aged - Acid Formation % | 8.3 | 59.0 | 109.6 | 3.5 | 52.8 |

### Comparative Example 18:

A 100 g comparative composition was prepared in the same manner as described in Example 6, with the exceptions that 95 g of petroleum jelly and 0 g of dimethyl isosorbide were used.

### Example 6:

A 100 g skin care composition was prepared by adding 90 g of petroleum jelly, 5 g of dimethyl isosorbide, and 5 g of glycolide into a mixing container and heating the components to 50 °C. When the petroleum jelly started to liquefy, the petroleum jelly, dimethyl isosorbide, and glycolide were mixed together at 50 °C using a Lightnin overhead mixer equipped with a 3-blade tilted paddle until a homogenous mixture was obtained. After a homogenous mixture was formed at 50 °C, the heat was removed and mixing continued until the homogenous mixture became too viscous to stir.

The homogenous mixture was then tested for skin penetration, the results of which are reported in Table 5.

### Example 7:

A 100 g skin care composition was prepared in the same manner as Example 6, with the exceptions that 85 g of petroleum jelly and 10 g of dimethyl isosorbide were used instead of 90 g and 5 g, respectively.

### Example 8:

A 100 g skin care composition was prepared in the same manner as Example 6, with the exceptions that 85 g of petroleum jelly and 10 g of glycolide were used instead of 90 g and 5 g, respectively.

### Example 9:

A 100 g skin care composition was prepared in the same manner as Example 6, with the exceptions that 80 g of petroleum jelly, 10 g of dimethyl isosorbide, and 10 g of glycolide were used instead of 90 g, 5 g, and 5 g, respectively.

**Table 5**

| Ingredient (wt. %) | Control | Comp. Ex. 18 | Example 6 | Example 7 | Example 8 | Example 9 |
|---|---|---|---|---|---|---|
| DMI | - | - | 5 | 10 | 5 | 10 |
| Petroleum Jelly | - | 95 | 90 | 85 | 85 | 80 |
| Glycolide | - | 5 | 5 | 5 | 10 | 10 |
| Water | 100 | - | - | - | - | - |
| | | | | | | |
| Control Layer | 0.08 | 0.02 | 0.08 | 0.15 | 0.13 | 0.05 |
| Surface Layer | 0.05 | 0.09 | 0.16 | 0.23 | 0.15 | 0.21 |
| Skin Layer 1 | 0.05 | 0.11 | 0.19 | 0.21 | 0.17 | 0.27 |
| Skin Layer 2 | 0.07 | 0.14 | 0.20 | 0.37 | 0.21 | 0.32 |
| Skin Layer 3 | 0.05 | 0.12 | 0.21 | 0.23 | 0.18 | 0.35 |
| Skin Layer 4 | 0.04 | 0.10 | 0.16 | 0.28 | 0.19 | 0.29 |
| Skin Layer 5 | 0.05 | 0.08 | 0.16 | 0.18 | 0.15 | 0.29 |
| Skin Layer 6 | 0.05 | 0.08 | 0.13 | 0.20 | 0.18 | 0.22 |
| Skin Layer 7 | 0.05 | 0.10 | 0.14 | 0.13 | 0.14 | 0.21 |
| Skin Layer 8 | 0.06 | 0.10 | 0.15 | 0.19 | 0.15 | 0.17 |
| Skin Layer 10 | 0.07 | 0.09 | 0.12 | 0.14 | 0.12 | 0.15 |

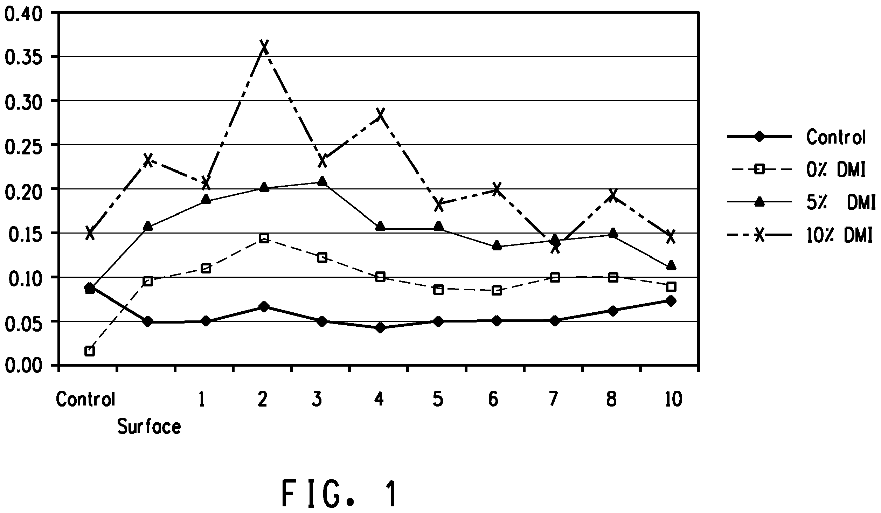

The skin penetration of Examples 6 - 9 are shown above in Table 5 and illustrated in Figs. 1 - 3. The absorbance for each skin layer was determined at 1766 cm-1, which is a region where glycolide absorbs well, while the non-cyclic dimer and glycolic acid absorb less. Accordingly, a skin layer demonstrating a higher absorbance value at 1766 cm-1 correlates to a higher concentration of glycolide at that skin layer. In this respect, the skin penetration properties can be determined by measuring the concentration of glycolide at various skin layers.

Examples 6 and 7, and Comparative Example 18, each contain 5 wt. % of glycolide. However, Examples 6 and 7, which have 5 wt. % and 10 wt. % of a polar non-aqueous solvent, respectively, show significantly better skin penetration properties versus Comparative Example 18, which does not have a polar non-aqueous solvent. See Fig. 1. Specifically, Examples 6 and 7 have skin penetration rates that are approximately double that of Comparative Example 18 at the skin surface and in the first several skin layers. Example 7 demonstrates significantly improved skin penetration even in deeper skin layers.

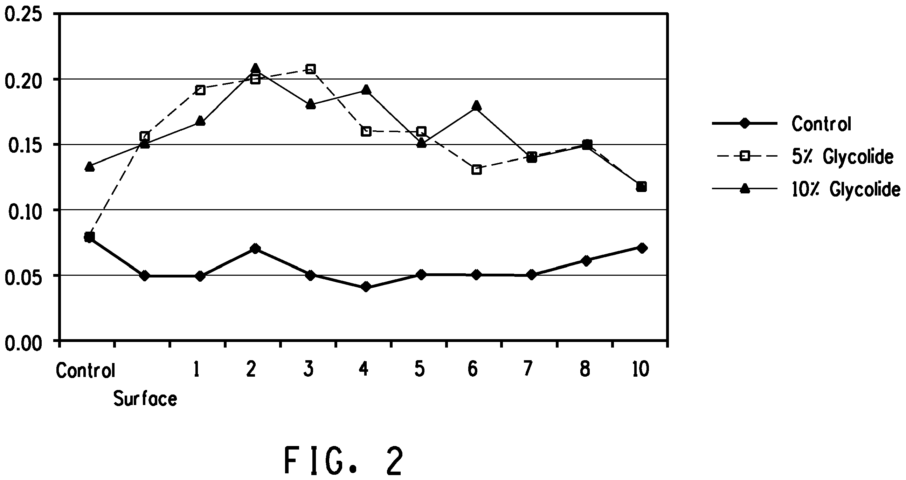

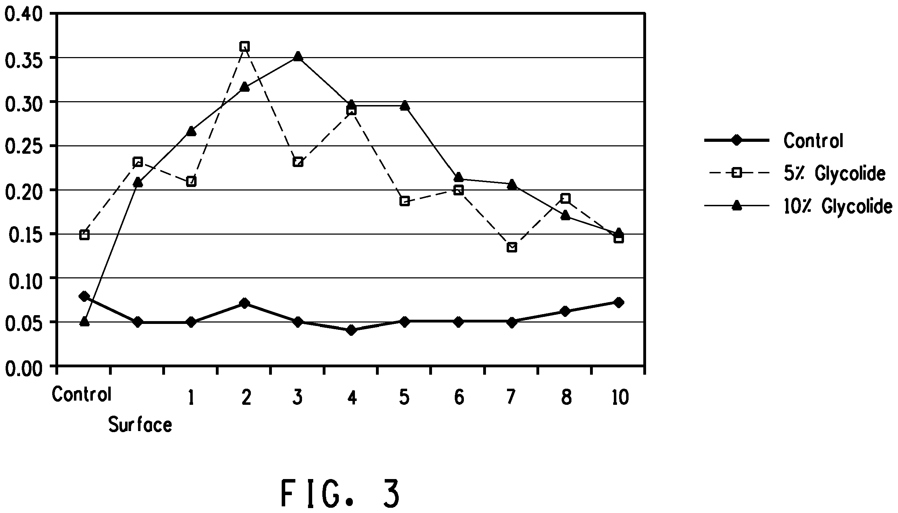

Examples 6 and 8, which have 5 wt. % and 10 wt. % of glycolide, respectively, show significantly improved skin penetration properties versus the control when the amount of a polar non-aqueous solvent is controlled at 5 wt. %. See Fig. 2. When the polar non-aqueous solvent is controlled at 10 wt. % in Examples 7 and 9, which have 5 wt. % and 10 wt. % of glycolide, respectively, significantly improved skin penetration properties versus the control are also shown. See Fig. 3.

### Example 10:

A 100 g skin care composition was prepared in the same manner as Example 9. The homogenous mixture was then tested for skin penetration, the results of which are reported in Table 6.

### Example 11:

A 100 g skin care composition was prepared in the same manner as Example 7. The homogenous mixture was then tested for skin penetration, the results of which are reported in Table 6.

### Comparative Example 19:

A 100 g comparative composition was prepared in the same manner as described in Example 6, with the exceptions that 99 g of petroleum jelly, 1 g of glycolide, and 0 g of dimethyl isosorbide were used.

### Comparative Example 20:

A 100 g comparative composition was prepared in the same manner as described in Comparative Example 6.

### Comparative Example 21:

A 100 g comparative composition was prepared in the same manner as described in Comparative Example 6 with the exception that 2 g of lactide was used instead of 2 g of glycolide.

**Table 6**

| Ingredient (wt. %) | Example 10 | Example 11 | Comp. Ex. 19 | Comp. Ex. 20 | Comp. Ex, 21 |
|---|---|---|---|---|---|
| DMI | 10 | 10 | - | - | - |
| Petroleum Jelly | 80 | 85 | 99 | 66 | 66 |
| Glycolide | 10 | 5 | 1 | 2 | - |
| Lactide | - | - | - | - | 2 |
| Mineral Oil | - | - | - | 32 | 32 |
| Water | - | - | - | - | - |
| | | | | | |
| Control Layer | -0.03 | 0.07 | 0.02 | 0.01 | 0.01 |
| Surface Layer | 0.16 | 0.18 | 0.03 | 0.01 | 0.01 |
| Skin Layer 1 | 0.22 | 0.16 | 0.08 | 0.02 | 0.01 |
| Skin Layer 2 | 0.25 | 0.3 | 0.05 | 0.01 | 0.01 |
| Skin Layer 3 | 0.3 | 0.18 | 0.07 | 0 | 0 |
| Skin Layer 4 | 0.25 | 0.24 | 0.08 | 0.01 | 0.01 |
| Skin Layer 5 | 0.24 | 0.13 | 0.06 | 0.01 | 0.01 |
| Skin Layer 6 | 0.17 | 0.15 | 0.06 | 0.01 | 0.01 |
| Skin Layer 7 | 0.16 | 0.08 | 0.05 | 0 | 0 |
| Skin Layer 8 | 0.11 | 0.13 | 0.06 | 0.01 | 0.01 |
| Skin Layer 10 | 0.08 | 0.07 | 0.02 | 0.01 | 0.01 |

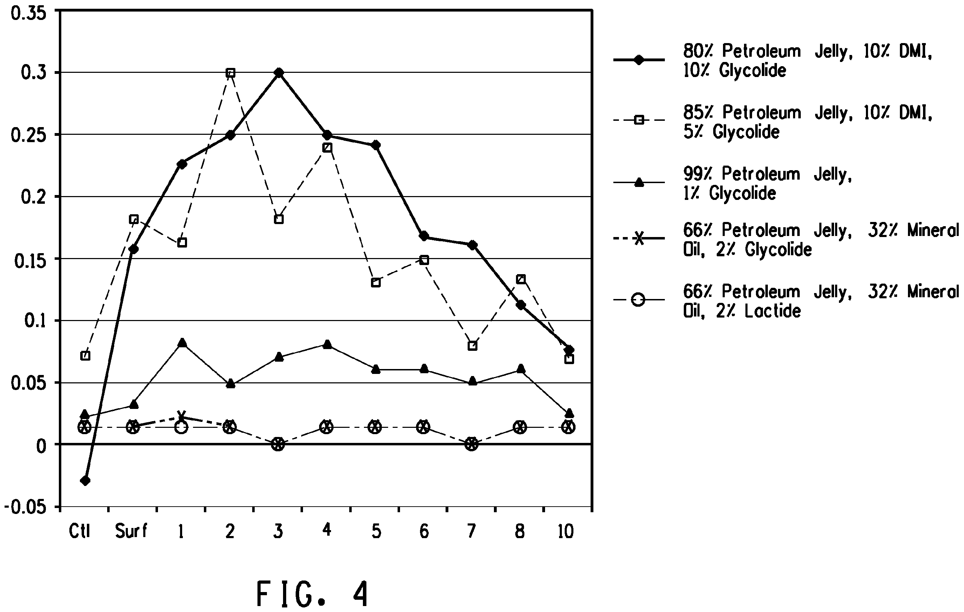

The normalized skin penetration data of Examples 10 and 11 are shown in Table 6 and illustrated in Fig. 4. The absorbance for each skin layer was determined at 1766 cm-1, which is a region where glycolide absorbs well, while the non-cyclic dimer and glycolic acid absorb less. Accordingly, a skin layer demonstrating a higher absorbance value at 1766 cm-1 correlates to a higher concentration of glycolide at that skin layer. In this respect, the skin penetration properties can be determined by measuring the concentration of glycolide at various skin layers.

Examples 10 and 11 each contain 10 wt. % of a polar non-aqueous solvent, and 10 wt. % and 5 wt. % of glycolide, respectively. Both Examples 10 and 11 have significantly better skin penetration properties versus Comparative Examples 19-21, which do not have a polar non-aqueous solvent. Specifically, when normalized, Examples 10 and 11 demonstrate better skin penetration properties in all the tested skin layers, especially at the skin surface and first several skin layers.

## Claims

1. Non-therapeutic Use of a composition as a skin care composition, wherein the composition comprises:
(a) 0.1 - 30 wt. % of at least one cyclic diester of an alpha hydroxy acid;
(b) 5 - 30 wt. % of at least one polar non-aqueous solvent having a polarity of 5 to 20; and
(c) at least 40 wt. % of at least one aliphatic base having a polarity of 5 or less;
the polarities being as defined in Hansen Solubility Parameters: A User's Handbook (2nd ed. 2007); and
wherein the composition comprises less than 1 wt. % of water.

2. The use of claim 1, wherein the cyclic diester is a C₄-C₈ diester dimer of two C₂-C₄ alpha hydroxy acids.

3. The use of claim 1, wherein the cyclic diester is glycolide, lactide, or mixtures thereof.

4. The use of claim 1, wherein the composition comprises 1 - 5 wt. % of the cyclic diester.

5. The use of claim 1, wherein the polar non-aqueous solvent has a polarity of 8 to 18.

6. The use of claim 1, wherein the composition comprises 7.5 - 15 wt. % of the polar non-aqueous solvent.

7. The use of claim 1, wherein the base has less than 0.5 wt. % of water.

8. The use of claim 1, wherein the base comprises at least one: (a) C₁₅-C₄₀ straight or branched alkane, alkene, or alkyne; (b) at least one cyclic aliphatic hydrocarbon, optionally substituted with at least one straight or branched C₁₀-C₃₅ straight or branched alkane, alkene, or alkyne; and (c) mixtures thereof.

9. The use of claim 1, wherein the base has a polarity of 2.5 or less.

10. The use of claim 1, wherein the composition comprises 0.5 wt. % or less of water.

11. The use of clam 1, wherein the composition comprises:
(a) 1 - 5 wt. % of glycolide, lactide, or combinations thereof;
(b) 10 - 20 wt. % of at least one polar non-aqueous solvent having a polarity of 7 to 11, a hydrogen bonding potential of 6 to 10, and having formula (IV): 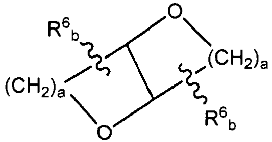 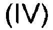 wherein R⁶ is selected from a C₁-C₅ alkoxy group, a C₁-C₅ aryloxy group, or combinations thereof; a is 1 to 3; and b is1 to 3; and
(c) at least 80 wt. % of at least one aliphatic hydrocarbon base having a polarity of 5 or less and a melting point greater than 25 °C;
the polarities being as defined in Hansen Solubility Parameters: A User's Handbook (2nd ed. 2007); and
wherein the composition comprises less than 1 wt. % of water.

12. The use of claim 11, wherein the polar non-aqueous solvent is isosorbide dimethyl ether.

## Patentansprüche

1. Nichttherapeutische Verwendung einer Zusammensetzung als eine Hautpflegezusammensetzung, wobei die Zusammensetzung umfasst:
(a) 0,1 bis 30 Gew.-% mindestens eines cyclischen Diesters einer alpha-Hydroxysäure;
(b) 5 bis 30 Gew.-% mindestens eines polaren nichtwässrigen Lösemittels, das eine Polarität von 5 bis 20 aufweist; und
(c) mindestens 40 Gew.-% mindestens einer aliphatischen Base, die eine Polarität von 5 oder weniger aufweist;
wobei die Polaritäten in Hansen Solubility Parameters: A User's Handbook (2. Ausg. 2007) festgelegt sind; und
wobei die Zusammensetzung weniger als 1 Gew.-% Wasser umfasst.

2. Verwendung nach Anspruch 1, wobei der cyclische Diester ein C₄-C₈-Diester-Dimer von zwei C₂-C₄-alpha-Hydroxysäuren ist.

3. Verwendung nach Anspruch 1, wobei der cyclische Diester ein Glykolid, Lactid oder Mischungen davon ist.

4. Verwendung nach Anspruch 1, wobei die Zusammensetzung 1 bis 5 Gew.-% des cyclischen Diesters umfasst.

5. Verwendung nach Anspruch 1, wobei das polare nichtwässrige Lösemittel eine Polarität von 8 bis 18 aufweist.

6. Verwendung nach Anspruch 1, wobei die Zusammensetzung 7,5 bis 15 Gew.-% des polaren nichtwässrigen Lösemittels aufweist.

7. Verwendung nach Anspruch 1, wobei die Base weniger als 0,5 Gew.-% Wasser aufweist.

8. Verwendung nach Anspruch 1, wobei die Base mindestens eines der folgenden umfasst: (a) unverzweigtes oder verzweigtes C₁₅-C₄₀-Alkan, -Alken oder -Alkin; (b) mindestens einen cyclischen aliphatischen Kohlenwasserstoff, gegebenenfalls substituiert mit mindestens einem unverzweigten oder verzweigten C₁₀-C₃₅-Alkan, -Alken oder-Alkin; und (c) Mischungen davon.

9. Verwendung nach Anspruch 1, wobei die Base eine Polarität von 2,5 oder weniger aufweist.

10. Verwendung nach Anspruch 1, wobei die Zusammensetzung 0,5 Gew.-% Wasser oder weniger aufweist.

11. Verwendung nach Anspruch 1, wobei die Zusammensetzung umfasst:
(a) 1 bis 5 Gew.-% Glykolid, Lactid oder Kombinationen davon aufweist;
(b) 10 bis 20 Gew.-% mindestens eines polaren nichtwässrigen Lösemittels, das eine Polarität von 7 bis 11 aufweist, ein Wasserstoffbindungspotential von 6 bis 10 und Formel (IV) aufweist: 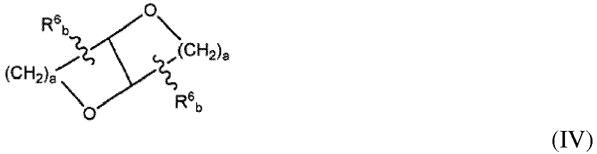 worin R⁶ ausgewählt ist aus einer C₁-C₅-Alkoxy-Gruppe, einer C₁-C₅-Aryloxy-Gruppe oder Kombinationen davon; a 1 bis 3 beträgt, und b 1 bis 3 beträgt; und
(c) mindestens 80 Gew.-% mindestens einer aliphatischen Kohlenwasserstoff-Base, die eine Polarität von 5 oder weniger und einen Schmelzpunkt von höher als 25 °C aufweist;
wobei die Polaritäten in Hansen Solubility Parameters: A User's Handbook (2. Ausg. 2007) festgelegt sind; und
wobei die Zusammensetzung weniger als 1 Gew.-% Wasser umfasst.

12. Verwendung nach Anspruch 11, wobei das polare nichtwässrige Lösemittel Isosorbid-dimethylether ist.

## Revendications

1. Utilisation non thérapeutique d'une composition en tant que composition de soins de la peau, dans laquelle la composition comprend:
(a) 0,1 à 30% en poids d'au moins un diester cyclique d'un alpha hydroxy acide;
(b) 5 à 30% en poids d'au moins un solvant polaire non aqueux ayant une polarité de 5 à 20; et
(c) au moins 40% en poids d'au moins une base aliphatique ayant une polarité de 5 ou moins;
les polarités étant telles que définies dans Hansen Solubility Parameters: A User's Handbook (2e éd. 2007); et
dans laquelle la composition comprend moins de 1% en poids d'eau.

2. Utilisation selon la revendication 1, dans laquelle le diester cyclique est un diester dimère en C₄-C₈ de deux alpha hydroxy acides en C₂-C₄.

3. Utilisation selon la revendication 1, dans laquelle le diester cyclique est un glycolide, un lactide ou des mélanges de ceux-ci.

4. Utilisation selon la revendication 1, dans laquelle la composition comprend 1 à 5% en poids du diester cyclique.

5. Utilisation selon la revendication 1, dans laquelle le solvant polaire non aqueux a une polarité de 8 à 18.

6. Utilisation selon la revendication 1, dans laquelle la composition comprend 7,5 à 15% en poids du solvant polaire non aqueux.

7. Utilisation selon la revendication 1, dans laquelle la base a moins de 0,5% en poids d'eau.

8. Utilisation selon la revendication 1, dans laquelle la base comprend au moins un: (a) alcane, alcène ou alcyne en C₁₅-C₄₀ linéaire ou ramifié; (b) au moins un hydrocarbure cyclique aliphatique, facultativement substitué par au moins un alcane, alcène ou alcyne en C₁₀-C₃₅ linéaire ou ramifié; et (c) des mélanges de ceux-ci.

9. Utilisation selon la revendication 1, dans laquelle la base a une polarité de 2,5 ou moins.

10. Utilisation selon la revendication 1, dans laquelle la composition comprend 0,5% en poids ou moins d'eau.

11. Utilisation selon la revendication 1, dans laquelle la composition comprend:
(a) 1 à 5% en poids de glycolide, de lactide ou de combinaisons de ceux-ci,
(b) 10 à 20% en poids d'au moins un solvant polaire non aqueux ayant une polarité de 7 à 11, un potentiel de liaison hydrogène de 6 à 10, et ayant la formule (IV): 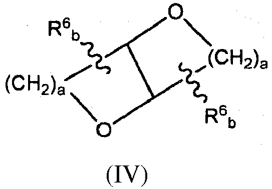 dans laquelle R⁶ est choisi parmi un groupe alcoxy en C₁-C₅, un groupe aryloxy en C₁-C₅, ou des combinaisons de ceux-ci; a est 1 à 3; et b est 1 à 3; et
(c) au moins 80% en poids d'au moins une base hydrocarbonée aliphatique ayant une polarité de 5 ou moins et un point de fusion supérieur à 25°C;
les polarités étant telles que définies dans Hansen Solubility Parameters: A User's Handbook (2e éd. 2007); et
dans laquelle la composition comprend moins de 1% en poids d'eau.

12. Utilisation selon la revendication 11, dans laquelle le solvant polaire non aqueux est l'éther diméthylique d'isosorbide.
